# EUROPEAN PATENT APPLICATION

(11) **EP 3 725 881 A1**
(43) Date of publication of application: **21.10.2020**
(21) Application number: 18888263.3
(22) Date of filing: 13.12.2018
(51) Int. Cl.: C12N 15/113, C12Q 1/68, G01N 33/50

(54) **METHOD FOR AIDING DETECTION OF PANCREATIC CANCER**

(30) Priority: 13.12.2017 JP 2017238839
(71) Applicant: Hiroshima University, Higashihiroshima-shi Hiroshima 739-8511 (JP)
(72) Inventor: TAHARA, Hidetoshi, Hiroshima-shi, Hiroshima 734-8553 (JP)
(74) Representative: Mewburn Ellis LLP
(86) International application number: PCT/JP2018/045993
(87) International publication number: WO 2019/117269

(57) **Abstract**

The present invention aims at providing a method of assisting the detection of pancreatic cancer with high accuracy. The present invention provides a method of assisting the detection of pancreatic cancer, which includes using as an index the abundance of at least one of miRNAs, isoform miRNAs (isomiRs), precursor miRNAs, transfer RNA fragments (tRFs), or non-coding RNA fragments (MiscRNAs) contained in a test sample isolated from a living body, whose nucleotide sequence is represented by any one of SEQ ID NOs: 1, 4, 6 to 10, 12, 15, 17, 19 to 21, 2, 3, 5, 11, 13, 14, 16, 18, and 22 to 38, wherein a higher abundance of at least one of the miRNAs, isomiRs, precursor miRNAs, or transfer RNA fragments whose nucleotide sequence is represented by any one of SEQ ID NOs: 1 to 20 than that of healthy subjects or a lower abundance of at least one of the miRNAs, isomiRs, precursor miRNAs, or non-coding RNA fragments (MiscRNAs) whose nucleotide sequence is represented by any one of SEQ ID NOs: 21 to 38 than that of healthy subjects indicates a higher likelihood of having pancreatic cancer.

SEQUENCE LISTING

<110>  HIROSHIMA UNIVERSITY

<120>  Method of aiding detection of pancreas cancer

<130>  PF632-PCT

<160>  38

<170>  PatentIn version 3.5

<210>  1
<211>  21
<212>  RNA
<213>  Homo sapiens

<400>  1
uuaugguuug ccugggacug a                                                21


<210>  2
<211>  19
<212>  RNA
<213>  Homo sapiens

<400>  2
uucauugcug ucggugggu                                                   19


<210>  3
<211>  18
<212>  RNA
<213>  Homo sapiens

<400>  3
ucauugcugu cggugggu                                                    18


<210>  4
<211>  23
<212>  RNA
<213>  Homo sapiens

<400>  4
ucaagagcaa uaacgaaaaa ugu                                              23


<210>  5
<211>  22
<212>  RNA
<213>  Homo sapiens

<400>  5
ucaagagcaa uaacgaaaaa ug                                               22


<210>  6
<211>  21
<212>  RNA
<213>  Homo sapiens

<400>  6

aaagcugggu ugagagggcg a                                                21

<210>  7
<211>  21
<212>  RNA
<213>  Homo sapiens

<400>  7
uggaguguga caaugguguu u                                                21

<210>  8
<211>  24
<212>  RNA
<213>  Homo sapiens

<400>  8
ugaggggcag agagcgagac uuuu                                             24

<210>  9
<211>  23
<212>  RNA
<213>  Homo sapiens

<400>  9
ugaggggcag agagcgagac uuu                                              23

<210>  10
<211>  22
<212>  RNA
<213>  Homo sapiens

<400>  10
cagugcaaug uuaaaagggc au                                               22

<210>  11
<211>  21
<212>  RNA
<213>  Homo sapiens

<400>  11
aacccguaga uccgaucuug u                                                21

<210>  12
<211>  21
<212>  RNA
<213>  Homo sapiens

<400>  12
cauuauuacu uuugguacgc g                                                21

<210>  13
<211>  25
<212>  RNA
<213>  Homo sapiens

<400>  13

gcauuggugg uucaguggua gaauu                                          25


<210>  14
<211>  23
<212>  RNA
<213>  Homo sapiens

<400>  14
ugagaacuga auuccauggg uug                                            23


<210>  15
<211>  21
<212>  RNA
<213>  Homo sapiens

<400>  15
ugagaacuga auuccauggg u                                              21


<210>  16
<211>  19
<212>  RNA
<213>  Homo sapiens

<400>  16
guccaguuuu cccaggaau                                                 19


<210>  17
<211>  22
<212>  RNA
<213>  Homo sapiens

<400>  17
ucaggcucag uccccucccg au                                             22


<210>  18
<211>  21
<212>  RNA
<213>  Homo sapiens

<400>  18
ucgaccggac cucgaccggc u                                              21


<210>  19
<211>  19
<212>  RNA
<213>  Homo sapiens

<400>  19
accccacucc ugguaccau                                                 19


<210>  20
<211>  20
<212>  RNA
<213>  Homo sapiens

<400>  20

cguaccguga guaauaaugc                                                    20

<210>  21
<211>  21
<212>  RNA
<213>  Homo sapiens

<400>  21
aaaccguuac cauuacugag u                                                  21

<210>  22
<211>  21
<212>  RNA
<213>  Homo sapiens

<400>  22
uaaagugcug acagugcaga u                                                  21

<210>  23
<211>  24
<212>  RNA
<213>  Homo sapiens

<400>  23
uguaaacauc cccgacugga agcu                                               24

<210>  24
<211>  21
<212>  RNA
<213>  Homo sapiens

<400>  24
uguaaacauc cccgacugga a                                                  21

<210>  25
<211>  23
<212>  RNA
<213>  Homo sapiens

<400>  25
caaagugcug uucgugcagg uag                                                23

<210>  26
<211>  32
<212>  RNA
<213>  Homo sapiens

<400>  26
uacaaccccc cacugcuaaa uuugacuggc uu                                      32

<210>  27
<211>  21
<212>  RNA
<213>  Homo sapiens

<400>  27

uaaggugcau cuagugcaga u                                                21

<210>  28
<211>  25
<212>  RNA
<213>  Homo sapiens

<400>  28
cccccacugc uaaauuugac uggcu                                            25

<210>  29
<211>  20
<212>  RNA
<213>  Homo sapiens

<400>  29
uacaguauag augauguacu                                                  20

<210>  30
<211>  33
<212>  RNA
<213>  Homo sapiens

<400>  30
uacaaccccc cacugcuaaa uuugacuggc uuu                                   33

<210>  31
<211>  23
<212>  RNA
<213>  Homo sapiens

<400>  31
uaaagugcug acagugcaga uag                                              23

<210>  32
<211>  22
<212>  RNA
<213>  Homo sapiens

<400>  32
guacaguacu gugauaacug aa                                               22

<210>  33
<211>  34
<212>  RNA
<213>  Homo sapiens

<400>  33
ggcugguccg augguagugg guuaucagaa cuua                                  34

<210>  34
<211>  21
<212>  RNA
<213>  Homo sapiens

<400>  34

caaagugcuu acagugcagg u                                                21

<210>  35
<211>  23
<212>  RNA
<213>  Homo sapiens

<400>  35
   cgcauccccu agggcauugg ugu                                             23

<210>  36
<211>  22
<212>  RNA
<213>  Homo sapiens

<400>  36
uaaggugcau cuagugcaga ua                                               22

<210>  37
<211>  18
<212>  RNA
<213>  Homo sapiens

<400>  37
ugugcaaauc caugcaaa                                                    18

<210>  38
<211>  22
<212>  RNA
<213>  Homo sapiens

<400>  38
aaaagugcuu acagugcagg ua                                               22

## Description

### TECHNICAL FIELD

The present invention relates to a method of assisting the detection of pancreatic cancer.

### BACKGROUND ART

Among various cancers, the incidence of pancreatic cancer has been increasing year by year. The westernization of eating habits is pointed out as a reason behind the increase. Pancreatic cancer has few early symptoms and are highly proliferative and invasive, which reflects that the annual number of deaths from pancreatic cancer is almost equal to the annual number of new pancreatic cancer cases, and currently causes a particularly low survival rate. Since pancreas is located deep in the abdomen, it is very difficult to detect pancreatic cancer by some examination methods, such as X-ray imaging.

Thus, methods in which the abundance of microRNA (hereinafter referred to as "miRNA") in plasma is used as an index to detect pancreatic cancer are proposed (Patent Documents 1 to 4).

### PRIOR ART DOCUMENTS

### Patent Documents

Patent Document 1: WO 2014/003053 A1
Patent Document 2: JP 2009-521952 T
Patent Document 3: JP 2009-528070 T
Patent Document 4: JP 2010-527235 T

### SUMMARY OF THE INVENTION

### PROBLEM TO BE SOLVED BY THE INVENTION

As described above, various miRNAs have been proposed as indexes for the detection of pancreatic cancer and, needless to say, it is advantageous if pancreatic cancer can be detected with higher accuracy.

Thus, an object of the present invention is to provide a method of assisting the detection of pancreatic cancer which assists in highly accurate detection of pancreatic cancer.

### MEANS FOR SOLVING THE PROBLEM

As a result of intensive study, the inventors newly found miRNAs, isoform miRNAs (isomiRs), precursor miRNAs, transfer RNA fragments (tRFs), and non-coding RNA fragments (MiscRNAs) which increase or decrease in abundance in pancreatic cancer, and discovered that use of those RNA molecules as indexes enables highly accurate detection of pancreatic cancer, and thereby completed the present invention.

That is, the present invention provides the followings.
(1) A method of assisting the detection of pancreatic cancer, using as an index the abundance of at least one of miRNAs, isoform miRNAs (isomiRs), precursor miRNAs, transfer RNA fragments (tRFs), or non-coding RNA fragments (MiscRNAs) contained in a test sample isolated from a living body, whose nucleotide sequence is represented by any one of SEQ ID NOs: 1, 4, 6 to 10, 12, 15, 17, 19 to 21, 2, 3, 5, 11, 13, 14, 16, 18, and 22 to 38, wherein a higher abundance of at least one of the miRNAs, isomiRs, precursor miRNAs, or transfer RNA fragments whose nucleotide sequence is represented by any one of SEQ ID NOs: 1 to 20 than that of healthy subjects or a lower abundance of at least one of the miRNAs, isomiRs, precursor miRNAs, or non-coding RNA fragments (MiscRNAs) whose nucleotide sequence is represented by any one of SEQ ID NOs: 21 to 38 than that of healthy subjects indicates a higher likelihood of having pancreatic cancer.
(2) The method according to (1), wherein the abundance of at least one of miRNAs, isomiRs, precursor miRNAs, or transfer RNA fragments whose nucleotide sequence is represented by any one of SEQ ID NOs: 1, 4, 6 to 10, 12, 15, 17, 19 to 21, 2, 3, 5, 11, 13, 14, 16, and 18 is used as an index.
(3) The method according to (2), wherein the abundance of at least one of miRNAs or isomiRs whose nucleotide sequence is represented by any one of SEQ ID NOs: 1, 4, 6 to 10, 12, 15, 17, 19, 20, and 21 is used as an index.
(4) The method according to (2) or (3), wherein the abundance of at least one of miRNAs or isomiRs whose nucleotide sequence is represented by any one of SEQ ID NOs: 1 to 4, 6, and 7 is used as an index.

### EFFECT OF THE INVENTION

By the method of the present invention, pancreatic cancer can be highly accurately and yet conveniently detected. Thus, the method of the present invention will greatly contribute to the detection of pancreatic cancer.

### MODE FOR CARRYING OUT THE INVENTION

As described above, the abundance of a specified miRNAs, isomiRs, precursor miRNAs, transfer RNA fragments (tRFs), or non-coding RNA fragments (MiscRNAs) (hereinafter sometimes referred to as "miRNAs or the like" for convenience) contained in a test sample isolated from a living body is used as an index in the method of the present invention. The nucleotide sequences of these miRNAs or the like are as shown in Sequence Listing. The list of miRNAs or the like used in the method of the present invention is presented in Tables 1-1 and 1-2 below.

**Table 1-1**

| SEQ ID NO: | Class | Archetype | Type | Length (nucleotides) | Sequence |
|---|---|---|---|---|---|
| 1 | isomiR | mir-584 | Mature 5' sub | 21 | uuaugguuugccugggacuga |
| 2 | isomiR | mir-181 b-1 //mir-181b-2 | Mature 5' sub | 19 | uucauugcugucggugggu |
| 3 | isomiR | mir-181 b-1//mir-181 b-2 | Mature 5' sub | 18 | ucauugcugucggugggu |
| 4 | miRNA | mir-335 | Mature 5' | 23 | ucaagagcaauaacgaaaaaugu |
| 5 | isomiR | mir-335 | Mature 5' sub | 22 | ucaagagcaauaacgaaaaaug |
| 6 | isomiR | mir-320a | Mature 3' sub | 21 | aaagcuggguugagagggcga |
| 7 | isomiR | mir-122 | Mature 5' sub | 21 | uggagugugacaaugguguuu |
| 8 | isomiR | mir-423 | Mature 5' super | 24 | ugaggggcagagagcgagacuuuu |
| 9 | miRNA | mir-423 | Mature 5' | 23 | ugaggggcagagagcgagacuuu |
| 10 | miRNA | mir-130a | Mature 3' | 22 | cagugcaauguuaaaagggcau |
| 11 | isomiR | mir-99a | Mature 5' sub | 21 | aacccguagauccgaucuugu |
| 12 | miRNA | mir-126 | Mature 5' | 21 | cauuauuacuuuugguacgcg |
| 13 | tRF | Homo_sapiens_tRNA-Gly-CCC-1-1// ···*1 | Exact | 25 | gcauuggugguucagugguagaauu |
| 14 | isomiR | mir-146a | Mature 5' super | 23 | ugagaacugaauuccauggguug |
| 15 | isomiR | mir-146a | Mature 5' sub | 21 | ugagaacugaauuccaugggu |
| 16 | isomiR | mir-145 | Mature 5' sub | 19 | guccaguuuucccaggaau |
| 17 | miRNA | mir-484 | Mature 5' | 22 | ucaggcucaguccccucccgau |
| 18 | miRNA | mir-1307 | Mature 5' | 21 | ucgaccggaccucgaccggcu |
| 19 | isomiR | mir-4286 | Mature 5' super | 19 | accccacuccugguaccau |
| 20 | isomiR | mir-126 | Mature 3' sub | 20 | cguaccgugaguaauaaugc |
| 21 | isomiR | mir-451a | Mature 5' sub | 21 | aaaccguuaccauuacugagu |
| 22 | miRNA | mir-106b | Mature 5' | 21 | uaaagugcugacapngcagau |
| 23 | isomiR | mir-30d | Mature 5' super | 24 | uguaaacauccccgacuggaagcu |
| 24 | isomiR | mir-30d | Mature 5' sub | 21 | uguaaacauccccgacuggaa |
| 25 | miRNA | mir-93 | Mature 5' | 23 | caaagugcuguucgugcagguag |
| 26 | MiscRNA | ENST00000516507.1 | Exact | 32 | uacaaccccccacugcuaaauuugacuggcuu |
| 27 | isomiR | mir-18a | Mature 5' sub | 21 | uaaggugcaucuagugcagau |
| 28 | MiscRNA | ENST00000363745.1//...*2 | Exact | 25 | cccccacugcuaaauuugacuggcu |

**Table 1-2**

| SEQ ID NO: | Class | Archetype | Type | Length (nucleotides) | Sequence |
|---|---|---|---|---|---|
| 29 | miRNA | mir-144 | Mature 3' | 20 | uacaguauagaugauguacu |
| 30 | MiscRNA | ENST00000516507.1 | Exact | 33 | uacaaccccccacugcuaaauuugacuggcuuu |
| 31 | isomiR | mir-106b | Mature 5' super | 23 | uaaagugcugacagugcagauag |
| 32 | isomiR | mir-101-1//mir-101-2 | Mature 3' super | 22 | guacaguacugugauaacugaa |
| 33 | MiscRNA | ENST00000577883.2//...*3 | Exact | 34 | ggcugguccgaiigguaguggguuaucagaacuua |
| 34 | isomiR | mir-17 | Mature 5' sub | 21 | caaagugcuuacagugcaggu |
| 35 | miRNA | mir-324 | Mature 5' | 23 | cgcauccccuagggcauuggugu |
| 36 | isomiR | mir-18a | Mature 5' sub | 22 | uaaggugcaucuagugcagaua |
| 37 | isomiR | mir-19b-1//mir-19b-2 | Mature 3' sub | 18 | ugugcaaauccaugcaaa |
| 38 | isomiR | mir-106a | Mature 5' sub | 22 | aaaagugcuuacagugcaggua |

| | | | | | |
|---|---|---|---|---|---|
| *1 :Homo_sapiens_tRNA-Gly-CCC-1-1//Homo_sapiens_tRNA-Gly-CCC-1-2//Homo_sapiens_tRNA-Gly-GCC-2-)1//Homo_sapiens tRNA-Cly-CCC-2-2// Homo_sapiens_tRNA-Gly-GCC-2-3//Homo_sapiens_tRNA-Gly-GCC-2-4//Homo_sapiens_tRNA-Gly-GCC-2-5//Homo_sapiens_tRNA-Gly-GCC-2-6//Ho mo_sapiens_tRNA-Cly-CCC-3-1//Homo_sapiens_tRNA-Cly-CCC-5-1 *2: ENST00000363745.1//ENST00000364409.1//ENST00000516507.1//ENST00000391107.1//ENST00000459254.1 *3:ENST00000577883.2//ENST00000577984.2//ENST00000516507.1//ENST00000481041.3//ENST00000579625.2//ENST00000365571.2//ENST000005 78877.2//ENST00000364908.1 | | | | | |

Among the miRNAs or the like, miRNAs or the like whose nucleotide sequences are represented by SEQ ID NOs: 1 to 21 (for example, "a miRNA or the like whose nucleotide sequence is represented by SEQ ID NO: 1" is hereinafter sometimes referred to simply as "a miRNA or the like represented by SEQ ID NO: 1" or "one represented by SEQ ID NO: 1" for convenience) are present in plasma. Among the miRNAs or the like, miRNAs or the like whose nucleotide sequences are represented by SEQ ID NOs: 22 to 38 are present in serum.

In all of those miRNAs or the like, the logarithm of the ratio of the abundance in plasma from patients with pancreatic cancer to the abundance in plasma from healthy subjects (represented by "log FC" which means the logarithm of FC (fold change) to base 2) is more than 2.0 in absolute value (that is, a ratio of not less than about 4 or not more than about 1/4), showing a statistical significance (*t*-test; *p* < 0.05).

The abundance of miRNAs or the like represented by SEQ ID NOs: 1 to 20 are higher in patients with pancreatic cancer than in healthy subjects, while the abundance of miRNAs or the like represented by SEQ ID NOs: 21 to 38 is lower in patients with pancreatic cancer than in healthy subjects.

Among those, the miRNAs or the like represented by SEQ ID NOs: 1 to 4, 6, and 7 have a log FC value of not less than 4.0 in absolute value and thus function as indexes with especially high sensitivity, and are preferable.

The accuracy of each cancer marker is indicated using the area under the ROC curve (AUC: Area Under Curve) as an index, and cancer markers with an AUC value of 0.7 or higher are generally considered effective. AUC values of 0.90 or higher, 0.97 or higher, 0.99 or higher, and 1.00 correspond to cancer markers with high accuracy, very high accuracy, quite high accuracy, and complete accuracy (with no false-positive and false-negative events), respectively. Thus. the AUC value of each cancer marker is likewise preferably 0.90, more preferably not less than 0.97, still more preferably not less than 0.99, and most preferably 1.00 in the present invention. The ones represented by SEQ ID NOs: 1, 4, 6 to 10, 12, 15, 17. and 19 to 21 have an AUC value of 1.00, as specifically described in Examples below, and are especially preferable.

The test sample is not specifically limited, provided that the test sample is a body fluid containing miRNAs; typically, it is preferable to use a blood sample (including plasma, serum, and whole blood). For the ones represented by SEQ ID NOs: 22 to 38, which are present in serum, it is simple and preferable to use serum or plasma as a test sample. The method of extracting total RNA in serum or plasma is well known and is specifically described in Examples below. The method of extracting total RNA from exosomes in serum or plasma is itself known and is specifically described in more detail in Examples below.

The abundance of each miRNA or the like is preferably measured (quantified) using a next-generation sequencer. Any instrument may be used and is not limited to a specific type of instrument, provided that the instrument determines sequences, similarly to next-generation sequencers. In the method of the present invention, as specifically described in Examples below, use of a next-generation sequencer is preferred over quantitative reverse-transcription PCR (qRT-PCR), which is widely used for quantification of miRNAs, to perform measurements from the viewpoint of accuracy because miRNAs or the like to be quantified include, for example, isomiRs, in which only one or more nucleotides are deleted from or added to the 5' and/or 3' ends of the original mature miRNAs thereof, and which should be distinguished from the original miRNAs when measured. Briefly, though details will be described specifically in Examples below, the quantification method can be performed as follows. When the RNA content in serum or plasma is constant, the number of reads for each isomiR or mature miRNA per million reads is considered as the measurement value, where the total counts of reads measured in a next-generation sequencing analysis of the RNA content are normalized to one million reads. When the RNA content in serum or plasma is variable in comparison with healthy subjects due to a disease, miRNAs showing little abundance variation in serum and plasma may be used. In cases where the abundance of miRNAs or the like in serum or plasma is measured, at least one miRNA selected from the group consisting of let-7g-5p, miR425-3p, and miR425-5p is preferably used as an internal control, which are miRNAs showing little abundance variation in serum and plasma.

The cut-off value for the abundance of each miRNA or the like for use in evaluation is preferably determined based on the presence or absence of a statistically significant difference (*t*-test; *p <* 0.05, preferably *p* < 0.01, more preferably *p* < 0.001) from healthy subjects with regard to the abundance of the miRNA or the like. Specifically, the value of log₂ read counts (the cut-off value) can be preferably determined for each miRNA or the like, for example, at which the false-positive rate is optimal (the lowest); for example, the cut-off values (the values of log₂ read counts) for several miRNAs or the like are as indicated in Table 2. The cut-off values indicated in Table 2 are only examples, and other values may be employed as cut-off values as long as those values are appropriate to determine statistically significant difference. Additionally, the optimal cut-off values vary among different populations of patients and healthy subjects from which data is collected. However, the cut-off values indicated in Table 2 with an interval of usually ±20%, particularly ±10%, may be set as cut-off values.

Moreover, a method of detecting the abundance of miRNAs or the like in a test sample from human suspected of having or affected with pancreatic cancer is also provided.

That is, a method of detecting the abundance of at least one of miRNAs, isoform miRNAs (isomiRs), precursor miRNAs, transfer RNA fragments (tRFs), or non-coding RNA fragments (MiscRNAs) whose nucleotide sequence is represented by any one of SEQ ID NOs: 1, 4, 6 to 10, 12, 15, 17, 19 to 21, 2, 3, 5, 11, 13, 14, 16, 18, and 22 to 38 in a test sample from human suspected of having or affected with pancreatic cancer is also provided, wherein the method includes the steps of:
collecting a blood sample from human; and
measuring the abundance of the miRNA(s), isomiR(s), precursor miRNA(s), transfer RNA fragment(s), or non-coding RNA fragment(s) in the blood sample by means of a next-generation sequencer or qRT-PCR,
wherein the abundance of at least one of the miRNAs, isomiRs, precursor miRNAs, or transfer RNA fragments whose nucleotide sequence is represented by any one of SEQ ID NOs: 1 to 20 is higher than that in healthy subjects, or the abundance of at least one of the miRNAs, isomiRs, precursor miRNAs, or non-coding RNA fragments (MiscRNAs) whose nucleotide sequence is represented by any one of SEQ ID NOs: 21 to 38 is lower than that in healthy subjects.

Additionally, in cases where the detection of pancreatic cancer is successfully achieved by the above-described method of the present invention, an effective amount of an anti-pancreatic cancer drug can be administered to patients in whom pancreatic cancer is detected, to treat the pancreatic cancer. Examples of the anti-pancreatic cancer drug can include gemcitabine, Folfirinox (combinational use of fluorouracil, levofolinate, irinolecan, and oxaliplatin), gemcitabine/nab-paclitaxel (Gem/nabPTX), and TS-1 (S1).

The present invention will be specifically described below by way of examples and comparative examples. Naturally, the present invention is not limited by the examples below.

### Examples 1 to 38

### 1. Materials and Methods

### (1) Clinical Samples

Plasma samples from 5 patients with pancreatic cancer and from 5 healthy subjects were used.

Serum samples from 75 patients with pancreatic cancer and from 111 healthy subjects were used.

### (2) Extraction of RNA in Plasma or Serum

Extraction of RNA in serum or plasma was performed using the miRNeasy Mini kit (QIAGEN).
1) Each frozen plasma or scrum sample was thawed and centrifuged at 10000 rpm for 5 minutes at room temperature to precipitate aggregated proteins and blood cell components.
2) To a new 1.5-mL tube, 200 µL of the supernatant was transferred.
3) To the tube, 1000 µL of the QIAzol Lysis Reagent was added and mixed thoroughly to denature protein components.
4) To the tube, 10 µL of 0.05 nM cel-miR-39 was added as a control RNA for RNA extraction, mixed by pipetting, and then left to stand at room temperature for 5 minutes.
5) To promote separation of the aqueous and organic solvent layers, 200 µL of chloroform was added to the tube, mixed thoroughly, and left to stand at room temperature for 3 minutes.
6) The tube was centrifuged at 12000 x g for 15 minutes at 4°C and 650 µL of the upper aqueous layer was transferred to a new 2-mL tube.
7) For the separation of RNA, 975 µL of 100% ethanol was added to the tube and mixed by pipetting.
8) To a miRNeasy Mini spin column (hereinafter referred to as column), 650 µL of the mixture in the step 7 was transferred, left to stand at room temperature for 1 minute, and then centrifuged at 8000 x g for 15 seconds at room temperature to allow RNA to be adsorbed on the filter of the column. The flow-through solution from the column was discarded.
9) The step 8 was repeated until the total volume of the solution of the step 7 was filtered through the column to allow all the RNA to be adsorbed on the filter.
10) To remove impurities attached on the filter, 650 µL of Buffer RWT was added to the column and centrifuged at 8000 x g for 15 seconds at room temperature. The flow-through solution from the column was discarded.
11) To clean the RNA adsorbed on the filter, 500 µL of Buffer RPE was added to the column and centrifuged at 8000 x g for 15 seconds at room temperature. The flow-through solution from the column was discarded.
12) To clean the RNA adsorbed on the filter, 500 µL of Buffer RPE was added to the column and centrifuged at 8000 x g for 2 minutes at room temperature. The flow-through solution from the column was discarded.
13) To completely remove any solution attached on the filter, the column was placed in a new 2-mL collection tube and centrifuged at 10000 x g for 1 minute at room temperature.
14) The column was placed into a 1.5-mL tube and 50 µL of RNase-free water was added thereto and left to stand at room temperature for 1 minute.
15) Centrifugation was performed at 8000 x g for 1 minute at room temperature to elute the RNA adsorbed on the filter. The eluted RNA was used in the following experiment without further purification and the remaining portion of the eluted RNA was stored at -80°C.

### (3) Quantification of miRNAs or the Like

The quantification of miRNAs or the like was performed as follows.

In cases where miRNAs or the like from, for example, two groups are quantified, extracellular vesicles (including exosomes) isolated by the same method are used to purify RNAs through the same method, from which cDNA libraries are prepared and then analyzed by next-generation sequencing. The next-generation sequencing analysis is not limited by a particular instrument, provided that the instrument determines sequences.

### (4) Calculation of Cut-off Value and AUC

Specifically, the cut-off value and the AUC were calculated from measurement results as follows.

The logistic regression analysis was carried out using the JMP Genomics 8 to draw the ROC curve and to calculate the AUC. Moreover, the value corresponding to a point on the ROC curve which was closest to the upper left corner of the ROC graph (sensitivity: 1.0, specificity: 1.0) was defined as the cut-off value.

### 2. Results

The results are presented in Tables 2-1 and 2-2.

**Table 2-1**

| Example | SEQ ID NO: | Class | Archetype | Type | Length (nucleotides) | Average in pancreatic cancer patients | Average in healthy subjects | log₂ FC | AUC | Cut-off value |
|---|---|---|---|---|---|---|---|---|---|---|
| Example 1 | I | isomiR | mir-584 | Mature 5' sub | 21 | 3989 | 162 | 4.6 | 1.000 | 11.21 |
| Example 2 | 2 | isomiR | mir-181b-1//mir-181b-2 | Mature 5' sub | 19 | 3274 | 143 | 4.5 | 0.960 | 8.34 |
| Example 3 | 3 | isomiR | mir-181b-1//mir-181b-2 | Mature 5' sub | 18 | 4807 | 267 | 4.2 | 0.840 | 12.65 |
| Example 4 | 4 | miRNA | mir-335 | Mature 5' | 23 | 5007 | 233 | 4.4 | 1.000 | 9.63 |
| Example 5 | 5 | isomiR | mir-335 | Mature 5' sub | 22 | 3481 | 227 | 3.9 | 0.980 | 8.85 |
| Example 6 | 6 | isomiR | mir-320a | Mature 3' sub | 21 | 2380 | 117 | 4.3 | 1.000 | 9.63 |
| Example 7 | 7 | isomiR | mir-122 | Mature 5' sub | 21 | 10981 | 630 | 4.1 | 1.000 | 11.05 |
| Example 8 | 8 | isomiR | mir-423 | Mature 5' super | 24 | 1365 | 114 | 3.6 | 1.000 | 8.92 |
| Example 9 | 9 | miRNA | mir-423 | Mature 5' | 23 | 9623 | 1114 | 3.1 | 1.000 | 11.21 |
| Example 10 | 10 | miRNA | mir-130a | Mature 3' | 22 | 6851 | 647 | 3.4 | 1.000 | 12.17 |
| Example 11 | 11 | isomiR | mir-99a | Mature 5' sub | 21 | 1694 | 207 | 3 | 0.920 | 10.01 |
| Example 12 | 12 | miRNA | mir-126 | Mature 5' | 21 | 17554 | 2520 | 2.8 | 1.000 | 12.90 |
| Example 13 | 13 | tRF | Homo_sapiens_tRNA-Gly-CCC-1-1// ...*] | Exact | 25 | 1706 | 268 | 2.7 | 0.920 | 10.09 |
| Example 14 | 14 | isomiR | mir-146a | Mature 5' super | 23 | 1360 | 252 | 2.4 | 0.880 | 8.51 |
| Example 15 | 15 | isomiR | mir-146a | Mature 5' sub | 21 | 2467 | 411 | 2.6 | 1.000 | 10.63 |
| Example 16 | 16 | isomiR | mir-145 | Mature 5' sub | 19 | 6590 | 1150 | 2.5 | 0.800 | 10.43 |
| Example 17 | 17 | miRNA | mir-484 | Mature 5' | 22 | 10667 | 1914 | 2.5 | 1.000 | 13.06 |
| Example 18 | 18 | miRNA | mir-1307 | Mature 5' | 21 | 2713 | 489 | 2.5 | 0.920 | 10.51 |
| Example 19 | 19 | isomiR | mir-4286 | Mature 5' super | 19 | 977 | 180 | 2.4 | 1.000 | 9.42 |
| Example 20 | 20 | isomiR | mir-126 | Mature 3' sub | 20 | 807 | 206 | 2 | 1.000 | 8.85 |
| Example 21 | 21 | isomiR | mir-451 a | Mature 5' sub | 21 | 50334 | 296017 | -2.6 | 1.000 | 17.88 |
| Example 22 | 22 | miRNA | mir-106b | Mature 5' | 21 | 133 | 460 | -2.12 | 0.812 | 7.36 |
| Example 23 | 23 | isomiR | mir-30d | Mature 5' super | 24 | 281 | 962 | -2.17 | 0.870 | 9.02 |

**Table 2-2**

| Example | SEQ ID NO: | Class | Archetype | Type | Length (nucleotides) | Average in pancreatic cancer patients | Average in healthy subjects | log₂ FC | AUC | Cut-off value |
|---|---|---|---|---|---|---|---|---|---|---|
| Example 24 | 24 | isomiR | mir-30d | Mature 5' sub | 21 | 209 | 534 | -2.33 | 0.820 | 8.14 |
| Example 25 | 25 | miRNA | mir-93 | Mature 5' | 23 | 919 | 2689 | -2.43 | 0.823 | 10.78 |
| Example 26 | 26 | MiscRNA | ENST00000516507.1 | Exact | 32 | 34 | 109 | -2.47 | 0.815 | 6.03 |
| Example 27 | 27 | isomiR | mir-18a | Mature 5' sub | 21 | 22 | 83 | -2.47 | 0.804 | 4.99 |
| Example 28 | 28 | MiscRNA | ENST00000363745.1//...*2 | Exact | 25 | 64 | 288 | -2.64 | 0.844 | 7.09 |
| Example 29 | 29 | miRNA | mir-144 | Mature 3' | 20 | 146 | 689 | -2.72 | 0.832 | 7.55 |
| Example 30 | 30 | MiscRNA | ENST00000516507.1 | Exact | 33 | 26 | 101 | -2.79 | 0.817 | 5.31 |
| Example 31 | 31 | isomiR | mir-106b | Mature 5' super | 23 | 27 | 67 | -2.82 | 0.803 | 4.43 |
| Example 32 | 32 | isomiR | mir-101-1/1mir-101-2 | Mature 3' super | 22 | 31 | 123 | -2.87 | 0.831 | 5.21 |
| Example 33 | 33 | MiscRNA | ENST00000577883.2//...*3 | Exact | 34 | 42 | 159 | -2.88 | 0.855 | 5.91 |
| Example 34 | 34 | isomiR | mir-17 | Mature 5' sub | 21 | 353 | 1377 | -2.91 | 0.805 | 9.07 |
| Example 35 | 35 | miRNA | mir-324 | Mature 5' | 23 | 9 | 39 | -2.94 | 0.855 | 4.06 |
| Example 36 | 36 | isomiR | mir-18a | Mature 5' sub | 22 | 68 | 244 | -3.06 | 0.831 | 6.9 |
| Example 37 | 37 | isomiR | mir-19b-1//mir-19b-2 | Mature 3' sub | 18 | 11 | 54 | -3.08 | 0.834 | 3.45 |
| Example 38 | 38 | isomiR | mir-106a | Mature 5' sub | 22 | 30 | 96 | -3.08 | 0.801 | 4.28 |

As seen in these results, the abundance of the miRNAs or the like represented by SEQ ID NOs: 1 to 20 was significantly higher in the patients with pancreatic cancer than that in the healthy subjects, and the abundance of the miRNAs or the like represented by SEQ ID NOs: 21 to 38 was significantly lower in the patients with pancreatic cancer than in the healthy subjects. It was indicated that pancreatic cancer was able to be detected with high accuracy by the method of the present invention (Examples 1 to 38). Moreover, all the p-values determined by *t*-test in Examples 1 to 38 were less than 0.05, indicating the effectiveness in detection of pancreatic cancer.

Moreover, the ones represented by SEQ ID NOs: 1, 4, 6 to 10, 12, 15, 17, 19, 20, and 21 were indicated to have an AUC value of 1.00, which are especially preferable.

## Claims

1. A method of assisting the detection of pancreatic cancer, using as an index the abundance of at least one of miRNAs, isoform miRNAs (isomiRs), precursor miRNAs, transfer RNA fragments (tRFs), or non-coding RNA fragments (MiscRNAs) contained in a test sample isolated from a living body, whose nucleotide sequence is represented by any one of SEQ ID NOs: 1, 4, 6 to 10, 12, 15, 17, 19 to 21, 2, 3, 5, 11, 13, 14, 16, 18, and 22 to 38, wherein a higher abundance of at least one of the miRNAs, isomiRs, precursor miRNAs, or transfer RNA fragments whose nucleotide sequence is represented by any one of SEQ ID NOs: 1 to 20 than that of healthy subjects or a lower abundance of at least one of the miRNAs, isomiRs, precursor miRNAs, or non-coding RNA fragments (MiscRNAs) whose nucleotide sequence is represented by any one of SEQ ID NOs: 21 to 38 than that of healthy subjects indicates a higher likelihood of having pancreatic cancer.

2. The method according to claim 1, wherein the abundance of at least one of miRNAs, isomiRs, precursor miRNAs, or transfer RNA fragments whose nucleotide sequence is represented by any one of SEQ ID NOs: 1, 4, 6 to 10, 12, 15, 17, 19 to 21, 2, 3, 5, 11, 13, 14, 16, and 18 is used as an index.

3. The method according to claim 2, wherein the abundance of at least one of miRNAs or isomiRs whose nucleotide sequence is represented by any one of SEQ ID NOs: 1, 4, 6 to 10, 12, 15, 17, 19, 20, and 21 is used as an index.

4. The method according to claim 2 or 3, wherein the abundance of at least one of miRNAs or isomiRs whose nucleotide sequence is represented by any one of SEQ ID NOs: 1 to 4, 6, and 7 is used as an index.
